# EUROPEAN PATENT SPECIFICATION

(11) **EP 2 874 608 B1**
(45) Date of publication and mention of the grant of the patent: **01.07.2020**
(21) Application number: 13747654.5
(22) Date of filing: 19.07.2013
(51) Int. Cl.: A61K 9/20, A61K 9/14, A61K 9/16

(54) **DRY GRANULATES OF MESOPOROUS SILICA POWDERS**
TROCKENGRANULATE AUS MESOPORÖSEN KIESELSÄUREPULVERN
GRANULÉS SECS DE POUDRES DE SILICE MÉSOPOREUSE

(30) Priority: 20.07.2012 GB 201212866
(43) Date of publication of application: 27.05.2015
(73) Proprietor: Formac Pharmaceuticals N.V., 3001 Heverlee (BE)
(72) Inventor: VAN SPEYBROEK, Michiel, 2070 Burcht (BE); VERHEYDEN, Loes, 3390 Tielt-Winge (BE)
(74) Representative: Paemen, Liesbet R.J.
(86) International application number: PCT/EP2013/065258
(87) International publication number: WO 2014/013044

(56) References cited:
- WO-A1-2012/072580
- US-A1- 2007 275 068
- US-A1- 2009 060 816
- US-A1- 2011 244 002
- US-A1- 2012 107 367
- HEIKKILÄ T ET AL: "Evaluation of Mesoporous TCPSi, MCM-41, SBA-15, and TUD-1 Materials as API Carriers for Oral Drug Delivery", DRUG DELIVERY, ACADEMIC PRESS, ORLANDO, FL, US, vol. 14, 1 January 2007 (2007-01-01), pages 337-347, XP008147597, ISSN: 1071-7544, DOI: 10.1080/10717540601098823

## Description

### Field of the Invention

This invention concerns dry granulates comprising unloaded or active ingredient-loaded ordered mesoporous silicas as well as oral dosage forms prepared therefrom.

### Background of the Invention

Ordered mesoporous silica (OMS) materials display an array of uniform mesopores of 2-50 nm in diameter and show good hydrothermal and chemical stability. Their relatively large pore volume and high specific surface area result in a very high adsorptive capacity, which is why interest in the use of OMS materials as oral drug delivery carriers is rapidly growing.

Many of the recently developed drugs suffer from poor aqueous solubility, which may lead to incomplete dissolution throughout the gastro-intestinal tract, resulting in low and variable oral bioavailability and therefore in variable clinical response. OMS materials show great potential to enhance the apparent solubility and dissolution rate of poorly water-soluble drugs and drug candidates. When a drug is entrapped inside the mesopores of an OMS material (the incorporation of the drug in the pores is usually conducted via solvent-based impregnation techniques), crystallization of the drug molecules is greatly hindered and essentially suppressed so that the drug is retained in its amorphous form. This suppression of crystallization is a result of the narrow diameter of the pores, which prevents the formation of crystal nuclei. OMS materials are therefore very effective at stabilizing the amorphous state of drugs. Amorphous formulations are widely used to overcome solubility-limited bioavailability, as the amorphous state has higher free energy and higher solubility than the crystalline state. In many cases however, amorphous drugs spontaneously convert back to the low-solubility crystalline state during processing or storage, which negatively impacts the pharmaceutical performance of the drug product. In this respect, OMS materials open up new possibilities as they permit robust stabilization of the amorphous state. A second effect that contributes to the dissolution-enhancement is the increase in the drug's surface area when adsorbed onto the OMS surface. The amorphous state of the confined drug and the increase in surface area available for contact with the dissolution medium offer the potential to greatly enhance apparent solubility and dissolution rate. Typically, when an OMS material loaded with a poorly water-soluble drug is exposed to an aqueous medium, the release rate from the OMS material is significantly faster than the dissolution rate of the crystalline drug.

US 6592764 discloses ordered mesoporous materials prepared using amphiphilic block copolymers, such as triblock polyoxyalkylenes, as templating agents in acidic media followed by calcination at high temperatures. One such material is SBA-15, a mesoporous silica material with an ordered, two-dimensional hexagonal (p6mm) honeycomb-like mesostructure.

EP 1852393 describes drug-loaded ordered mesoporous materials suited for immediate release applications.

WO 2009/133100 discloses ordered mesoporous materials prepared under slightly acidic or neutral conditions and their use as vehicles for drug release.

Van den Mooter et al. in Journal of Pharmaceutical Sciences (2011), Vol. 100(8), pp. 3411-3420 and in WO2012/072580 describe that the pressure required to compress active ingredient-loaded silica materials into a tablet negatively affected the performance of such materials in that a reduced drug release of the active ingredient was observed. These negative effects on release rate were avoided by adding microcrystalline cellulose and croscarmellose sodium that acted to protect the OMS material from pore collapse upon compression.

US 2011/244002 describes formulations comprising mesoporous silica materials and poorly aqueous soluble active ingredients. In § 0085 mention is made of granules but these are prepared by wet granulation, i.e. by addition of water or a suitable solvent.

US 2009/060816 discloses processes for producing fine mesoporous silica particles by wet pulverizing mesoporous silica particles while a surfactant is present in the mesopores of the silica particles; they can be used as carrier for medicines. In § 0098 mention is made of the possibility to granulate the mesoporous silica particles. No mention is made of dry granulation and of the possibility of dry granulates containing large amounts of mesoporous silica.

WO 2012/072580 concerns compressed formulations comprising ordered silica loaded with an active ingredient in combination with microcrystalline cellulose and croscarmellose sodium. The possibility of dry granulates containing large amounts of mesoporous silica is not mentioned in this document.

Most oral pharmaceutical products are supplied as solid dosage forms such as tablets or capsules, and the ability to produce stable and bioavailable solid dosage forms is therefore a key requirement for a new drug delivery technology. The capability to manufacture capsules and tablets on clinical and commercial scale depends critically on the flowability of the powder blend to be encapsulated/tableted. Flowability refers to the ability of a powder to flow out of a bin or hopper and through chutes or pipes, and depends on many factors such as the bulk density of the powder and the size and the shape of the powder particles. Friction between the powder particles and the hopper walls may also impair flowability. Good flowability is an essential requirement for capsule filling and tablet compression. Powders should reproducibly flow into the capsule shell or die of the tablet machine to ensure reproducible content uniformity, disintegration and dissolution. Flowability is a critical quality attribute of a powder to be encapsulated or tableted. A generally accepted way of assessing powder flow with small sample volumes is using the Carr Index (also: the Compressibility Index or Carr's Compressibility Index). The Carr index (CI) is calculated by the formula CI = 100 x (1-ρ_{b}/ρₜ), where ρ_{b} is the freely settled bulk density of a powder, and ρₜ is the tapped density of the powder. Table 1 ranks the flow characteristics in relation to the Carr Index. A Carr index greater than 25 is considered to be indicative of poor flowability.

**Table 1: Classification of powder flow using the Carr Index as described in the United States Pharmacopeia chapter <1174> Powder Flow**

| Carr Index (%) | Flow Character |
|---|---|
| ≤ 10 | Excellent |
| 11-15 | Good |
| 16-20 | Fair |
| 21-25 | Passable |
| 26-31 | Poor |
| 32-37 | Very poor |
| ≥ 38 | Very, very poor |

The properties of OMS materials as manufactured present substantial flowability challenges. Due to their porous nature, OMS materials (drug-loaded as well as unloaded) have a very low density. In addition, particles of such materials are small and in most cases non-spherical in shape. The combination of low bulk density, small particle size and non-spherical morphology explain why both drug-loaded and unloaded OMS materials exhibit very poor flowability. This makes these materials unapt for large scale processing such as in direct compression.

Flow characteristics and compaction properties of pharmaceutical powder blends can be improved by converting fine particles into larger, multiparticulate entities - a process known as granulation. Granulation processes can be subdivided in two classes: wet granulation and dry granulation. In wet granulation, granules are formed by blending powders in the presence of a granulation liquid, typically water or alcohols in which a binder, typically a polymer, is dissolved or dispersed. Granule formation occurs through the formation of liquid bridges between the primary particles. After granulation, the liquid phase is removed by drying, giving rise to granules that consist of the primary powder particles held together by the binder.

In dry granulation no liquids are used, for example because the product to be granulated is sensitive to moisture. In this process the powder particles are compressed, which increases the contact surface area between the powder particles thereby promoting the formation of interparticle bonds. Usually pressure is in itself insufficient to induce adequate particle binding, in which case a binder (e.g. microcrystalline cellulose) may be incorporated in the powder blend. Other excipients that may be included in the powder blends include disintegrants (e.g. croscarmellose), fillers (e.g. lactose) and lubricants (e.g. magnesium stearate).

Dry granulation can be conducted via two processes. In one process known as slugging, the powder blend is compressed in an eccentric or rotary compression machine to produce large tablets (slugs), which are subsequently milled and sieved to obtain granules of a certain particle size. In a second process known as roller compaction, the powder blend is fed through two rolls that rotate in counter clockwise direction, to produce sheet- or ribbon-like compacts that are milled and sieved to the desired particle size. Sometimes the process may require repeated compaction steps to attain the proper granular end point.

In particular in roller compaction a percentage of the powder product usually does not get compacted and upon comminuting, the resulting granulate may have too large a portion of fines. In that case, the resulting granulate will not have the required properties such as, for example, the flow characteristics required for proper processing into tablets. In that instance a sieving process may be required to remove excessive fines, which may be reprocessed in a further compaction step. In some instances it will not be necessary to compact the complete blend and the obtained granules can be blended back in with the rest of the formula's ingredients.

Lubricants may be added to avoid the material(s) being compressed from sticking to the punches. Commonly used lubricants include magnesium stearate, calcium stearate and sodium stearyl fumarate. Such lubricants are typically included in such amount that the concentration in the final tableted product is less than 1% by weight. Binders may also be added to improve interparticle adhesion. Also disintegrants may be added. Lubricants, binders and disintegrants may be added prior to, after granulation, or both.

In the case of drug-loaded OMS materials, dry granulation is preferred over wet granulation, as contact with water or other solvent systems used in wet granulation may lead to extraction of drug from the OMS pores, which in turn may lead to drug crystallisation and reduced pharmaceutical performance. Although dry granulation avoids this solvent-related risk of drug crystallisation, it presents a different challenge in that pressure may lead to collapse of the OMS pores. Pore collapse may in turn negatively affect the active ingredient-loading capacity, the release characteristics and the physical stability of OMS dry granules. Dry granulation of OMS materials therefore has to be conducted at relatively low pressures, which is unfavorable for obtaining adequate particle binding so that binders need to be added in compensation.

It is however important to minimize the level of additional excipients (e.g. binders and disintegrants) in a dry granulation powder blend to minimize the size of the final dosage form. Patients in general dislike having to swallow too large dosage forms, which in particular is the case in certain patient groups such as children or the elderly. This contributes to the patient's comfort when taking the prescribed medicine, and therefore to therapeutic compliance. Where larger amounts of active ingredient need to be administered, minimizing the size of the oral dosage forms avoids the need of multiple administrations, ideally leading to a drug product that requires intake of only one single capsule or tablet per administration.

There is therefore a need to provide dry granulates of OMS materials with good flowability characteristics and with limited quantities of additional excipients. There is an additional need to provide oral dosage forms such as tablets or capsules, based on drug-loaded dry granulates of OMS materials from which drug release is within acceptable boundaries.

### Summary of the Invention

It now has been found that OMS dry granulates as well as active ingredient-loaded OMS dry granulates with limited bulk and improved flowability can be obtained by dry granulation using a limited amount of additional excipients. The mixture of OMS and excipients or the mixture of active ingredient-loaded OMS and excipients is first compacted and then broken up, e.g. by milling. Dry granulation of OMS or of active ingredient-loaded OMS in accordance with the present invention can be conducted at relatively low pressures without the need to add large quantities of ingredients to improve particle adhesion such as binders. The resulting granulates contain limited amounts of additional excipients. The porosity of the OMS is kept intact and the physical stabilization and the release of active ingredients are maintained.

The dry granulation processes of the invention lead to improved flowability, with minimal dilution (minimal addition of other excipients). The release characteristics from dry granules prepared from OMS materials loaded with active ingredient or from dry granules prepared from unloaded OMS, having been subsequently loaded with an active ingredient, are within acceptable boundaries. Compared to non-granulated active ingredient-loaded OMS materials, the release characteristics are retained, in particular immediate release characteristics are retained. In the latter instance, dry granulated drug-loaded OMS materials are suitable for immediate release dosage forms.

Thus, in one aspect, the present invention is concerned with a dry granulate comprising from 50% to 100% w/w of ordered mesoporous silica having a hexagonally-ordered two-dimensional array of uniform sized cylindrical pores of 4 to 14 nm, or 5 - 14 nm, in particular 6 - 14 nm; a pore volume in the range from 0.8 - 1.2 mL/g, or 0.9 - 1.1 mL/g, and a specific surface area in the range from about 500 - 1000 m²/g, or from 700 - 1000 m²/g, wherein said ordered mesoporous silica is loaded with a biologically active ingredient having a molecular weight in the range of about 200 to about 1,000 (daltons), in particular in the range of about 200 to about 800 and optional excipients up to 100% w/w, said dry granulate having a Carr index of equal or lower than 25. The optional excipients may comprise one or more of a disintegrant, a binder, a lubricant, a filler, or other excipients. In particular embodiments, the dry granulate contains from 3 to 30 % of a binder. In particular embodiments, the dry granulate contains from 1 to 10 % of a disintegrant. In particular embodiments, the dry granulate contains from 0.5 to 2 % of a lubricant.

In particular embodiments of the dry granulate envisaged herein the ordered mesoporous silica is loaded with a poorly soluble biologically active ingredient.

In particular embodiments of the dry granulate envisaged herein the ordered mesoporous silica is a hexagonally-ordered two-dimensional array of uniform sized cylindrical pores of 6 to 10 nm.

This invention also concerns a process for preparing a dry granulate, such as those specified herein, wherein a mixture of OMS loaded with a pharmaceutically active ingredient and optional excipients is subjected to pressure. The compaction may be achieved by slugging using appropriate dies or by roller-compaction, both followed by milling of the compacted material into granules. The thus prepared granules may be sieved to select the desired particle size range.

In one embodiment, the dry granulate prepared from unloaded OMS material is subsequently loaded with an active ingredient.

In particular embodiments, the process comprises subjecting a mixture of ordered mesoporous silica loaded with a biologically active ingredient and optional excipients to pressure that is in the range of 30 to 50 MPa. In particular embodiments, the process comprises a step of compaction by 30 compression using dies thus forming a tablet, or by roller-compaction, optionally followed by milling of the compacted material. In particular embodiments of the process, the thus prepared dry granulate is sieved into a desired particle size range.

In still a further aspect, the present invention concerns a solid dosage form comprising the dry granulate as specified herein.

In one embodiment the solid dosage form is a tablet. In further embodiments the tablet is obtained by compressing a mixture of dry granulate, as specified herein, with one or more excipients. In one embodiment, the latter may be selected from croscarmellose sodium, microcrystalline cellulose, lactose and magnesium stearate, or combinations thereof.

In another embodiment, the solid dosage form is a capsule that is filled with dry granulate as envisaged herein, wherein the dry granulate is optionally loaded with an active ingredient. The dry granulate, the active ingredient as well as the loading may be as specified herein.

In one embodiment, the composition is a pharmaceutical composition.

In particular embodiments, the solid dosage form envisaged herein is for immediate release.

Accordingly, the application also provides solid dosage forms of the dry granulate loaded with an active ingredient as described herein for use in a method of treatment of a disease, wherein the method requires immediate release of said active ingredient.

### Description of the Figures

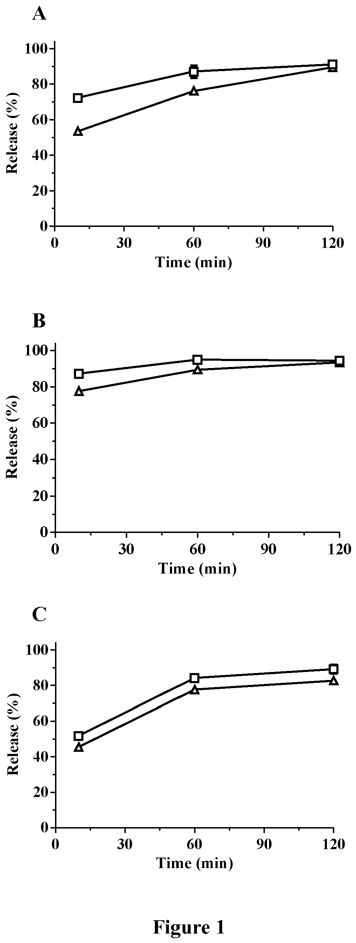

Figure 1: In vitro release profiles (*n*=3; mean ± standard deviation) from uncompressed loaded OMS-7 powders (□) and granules (Δ); % release of the drug in solution in function of time; OMS-7 is described in example 1; (A) Itraconazole recorded in 0.01 N HCl supplemented with 1% of sodium lauryl sulfate. (B) Fenofibrate recorded in 50 mM phosphate buffer supplemented with 25 mM of sodium lauryl sulfate. (C) Carbamazepine recorded in 50 mM phosphate buffer supplemented with 0.5% of sodium lauryl sulfate.

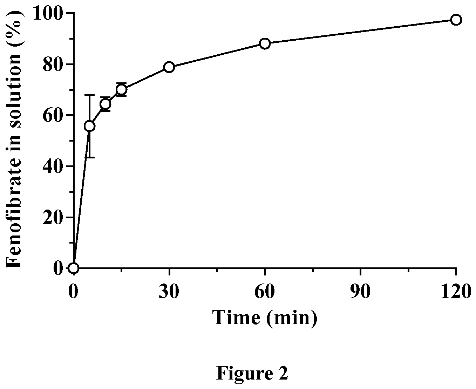

Figure 2: In vitro release profile of granules prepared from fenofibrate-loaded OMS7; % release of fenofibrate in solution (n=3; mean ± standard deviation) in function of time.

### Further Description of the Invention

As used herein, the singular is meant to include the plural and vice versa, the plural is meant to include the singular. For example the term "a" includes "one or more" and the term "one or more" includes "a". Any feature described in relation to a particular aspect or embodiment is meant to be applicable to one or more of the other aspects or embodiments described herein.

The terms "pharmaceutical active ingredient" and "drug" are meant to be equivalent. Drugs can be for human or for veterinary use. Pharmaceutical active ingredients comprise synthetic molecules, biomolecules, antibodies, and the like.

The terms "tablet" or "tablet formulation" refer to any compressed solid dosage form for administration of an active ingredient to a human or warm-blooded animal. Tablets may be for administration orally, sublingually, rectally, vaginally, or by implantation. They may take any shape or size known in the pharmaceutical art, such as round, oblong, capsule-shaped, or any other known form, and include caplets, minitablets, microtablets, and the like. If desired, a tablet may be covered with a coating.

The terms "mesopore" or "mesoporous" and the like refer to porous structures having pore sizes in the range of 2 nm to 50 nm. No particular spatial organization or method of manufacture is implied by these terms. Particular mesoporous silicas have pore sizes in the range of 2 nm to 30 nm, or in the range of 2 nm to 20 nm, or in the range of 4 nm to 16 nm, or in the range of 6 nm to 14 nm, or in the range of 5 nm to 12 nm.

Whenever used herein, the term "ordered" in relation to mesoporous silicas refers to silicas having at least one level of structural order (e.g. one or two), in particular having one level of structural order. In one embodiment the term "ordered" refers to ordered arrays of mesopores with regular pore size and morphology.

As used herein in relation to percentages, "w/w" means weight/weight, "w/v" means weight/volume.

An unloaded OMS or an active-ingredient loaded OMS is a very low density material that shows very poor flow properties making it difficult to handle and process. The Carr index of such materials is always over 25 and typically is over 35.

In accordance with the present invention unloaded OMS or active-ingredient loaded OMS is compacted to form a dry granulate, in particular compacted to about 1.5 to 3 times, in particular to about two times, its original tapped density.

The mixture of OMS and excipients or the mixture of active ingredient-loaded OMS and excipients may be first compacted by slugging to form large tablets (slugs) or by roller compaction to form plates or sheets. In this compaction step the mixture is subjected to a pressure that may be in the range of 20 to 300 MPa, in particular in the range of 20-100 MPa, preferably in the range of 30-50 MPa, more preferably in the range of 30-40 MPa, e.g. about 35 MPa. In the instance of roller compaction, the mechanical stress applied to the powder may also be expressed as the specific compaction force, i.e. the compaction force per length unit of roll width. Said force may be in the range of 1 to 10 kN/cm, or 2 to 5 kN/cm. The compacts are then broken up, e.g. by milling. The compacting may be conducted in an eccentric or rotary tablet press or in a roller compactor. The resulting dry granulate may be sieved to result in a dry granulate in the form of a powder of desired particle size.

In an alternative aspect, the present invention is concerned with a dry granulate comprising from 50% to 100% w/w of ordered mesoporous silica optionally loaded with a biologically active ingredient and optional excipients up to 100% w/w, said dry granulate having a tapped density of at least 0.3 g/mL. In certain embodiments, the tapped density may be at least 0.4 g/mL, or may be at least 0.5 g/mL. In one embodiment, the dry granulate of the invention may have a density that is lower than 0.8 g/mL, or lower than 0.7 g/mL, or lower than 0.6 g/mL. In certain embodiments, the dry granulate of the invention may have a density that is in the range of the minimal and maximal tapped densities mentioned herein, in particular in the range from 0.3 g/mL to 0.8 g/mL, or from 0.4 g/mL to 0.6 g/mL.

The dry granulate of the invention may comprise from 50 % to 100% w/w of ordered mesoporous silica loaded with a biologically active ingredient. In one embodiment, the dry granulate of the invention may comprise from 60 % to 90% w/w, or from 70 % to 90%, or from 75 % to 85%, for example about 80%, of ordered mesoporous silica loaded with a biologically active ingredient and optional excipients up to 100%, wherein any percentages in this paragraph are w/w.

The dry granulates of the invention in addition to the loaded OMS may comprise one or more optional excipients, which may be selected from one or more of a binder, a disintegrant, a filler, a lubricant and other excipients.

The binder may be present in the dry granulate in an amount that is in the range of from 5 to 50%, or from 10 to 30%, or from 15 to 20%, for example about 15%. Preferably the binder may be present in the dry granulate in an amount that is in the range of from 2 to 30%, or from 3 to 30%, or from 3 to 20%, or from 3 to 10%. The disintegrant may be present in the free-flowing compacted powder in an amount that is in the range of from 0 to 20%, or from 0.5 to 15%, or from 1 to 10 %, or from 2 to 8%, for example about 5%. The lubricant may be present in an amount that is in the range of 0 to 3%, or of 0 to 2%, or of 0.5 to 2%, for example about 1%. The filler may be present in an amount that is in the range of from 0 to 30%, or from 0 to 20%, or from 10 to 30 %. Other excipients may be present in amounts that complete 100%. All percentages in this paragraph are w/w.

It has been found that the dry granulates of the invention containing a binder, in particular in an amount as specified herein, show good results in terms of cohesion and flow characteristics. Such granulates may have a Carr index below 25, or below 20, or lower.

In addition it has been found that the dry granulates of the invention containing a disintegrant, in particular in an amount as specified herein, show good results in terms of release characteristics of the active ingredient loaded on or in the mesoporous silica. Such granulates may show immediate release of the active ingredient with release characteristics as specified further herein.

Binders that can be used include microcrystalline cellulose itself, modified microcrystalline cellulose and mixtures of microcrystalline cellulose with other ingredients, such as silicified microcrystalline cellulose. In one embodiment, the binder is microcrystalline cellulose.

Examples of microcrystalline cellulose products that can be used include:
- the Avicel™ series of products available from FMC BioPolymer, in particular Avicel DG™; Avicel PH 10™, Avicel PH 102™; Avicel PH 105™, Avicel PH 301™; Avicel PH 302™
- the microcrystalline cellulose products available from JRS Pharma, in particular Vivapur™ 105, Vivapur™ 101, Emcocel™ SP 15, Emcocel™ 50M 105, Prosolv™ SMCC 50, in particular Prosolv™ SMCC HD90
- the microcrystalline cellulose products available from DFE under the tradename Pharmacel™, in particular Pharmacel™105, Pharmacel™101
- the microcrystalline cellulose products available from Blanver, in particular Tabulose (Microcel)™101, Tabulose (Microcel)™103
- the microcrystalline cellulose products available from Asahi Kasei Corporation, such as Ceolus™ PH-F20JP, Ceolus™ PH-101, Ceolus™ PH-301, Ceolus™ KG-802, Ceolus™ KG- 1000.

Disintegrants that can be used include:
- the crosslinked povidones available from BASF, such as Kollidon™ CL, Kollidon™ CL-M, Polyplasdone™ XL, Polyplasdone™ XL-M
- the croscarmellose sodium products such as Ac-di-sol™ from FMC Biopolymer
- the sodium starch glycolate products such as Explotab™ from JRS Pharma

In one embodiment, the disintegrant is croscarmellose sodium.

Fillers that can be used include:
- lactose, in particular the materials available under the trade name Pharmatose™, or Supertab™21AN, Supertab™24AN, Supertab™11SD
- dibasic calcium phosphate, calcium carbonate, sucrose, glucose, mannitol, sorbitol In one embodiment, the filler is lactose.

Lubricants that can be used include calcium stearate, sodium stearyl fumarate or, preferably, magnesium stearate.

Various OMS materials may be used in the invention. OMS materials that can be used are those prepared by polymerizing a silica precursor such as tetraethylorthosilicate in the presence of a template, for example a quaternary ammonium salt such as cetyltrimethylammonium bromide or a non-ionic surfactant such as a poly(ethylene oxide)-poly(propylene oxide)-poly(ethylene oxide) block copolymer.

OMS materials can be obtained by linking silica precursors using structure directing organic template molecules such as surfactants or block copolymers, followed by removal of the structure directing molecules. Silica precursors include silicates such as alkali silicates, e.g. sodium silicate; silicic acids; and tetraalkyl orthosilicates, e.g. tetraethyl orthosilicate (TEOS), tetramethyl orthosilicate (TMOS) and tetrapropyl orthosilicate (TPOS). Silica precursors may also be derived from polymeric forms of silica through depolymerization. Examples include pyrogenic silica, silica gel and precipitated silica. The template molecules usually are removed by a calcination step at increased temperatures, such as above 400°C, or above 500°C, e.g. at 500°C, or alternatively by extraction using solvents that dissolve the structure directing organic template molecules.

Of interest are hexagonally ordered mesoporous silicas that have a honeycomb structure, i.e. the two-dimensional (2D)-hexagonal (plane group, p6mm) mesoporous silicas. Of interest are ordered mesoporous silicas having amorphous walls of sufficient thickness as to provide adequate structural integrity. In one embodiment, the walls have a thickness that is in the range of about 2 to about 14 nm, or about 2 to about 10 nm, or about 3 to about 10 nm, or about 3 to about 8 nm, or about 4 to about 8 nm, or about 4 to about 7 nm. The ordered mesoporous silicas for use in the present invention may have a pore diameter that is in the range of 5-20 nm, or in the range of 4-20 nm, or in the range of 5-16 nm, or in the range of 5-14 nm, or in the range of 6-20 nm, or in the range of 6-16 nm, or in the range of 6-14 nm. In one embodiment, the ordered mesoporous silicas do not have functionalized hydroxyl groups in the pores.

Ordered mesoporous silicas that can be used in the invention include the mesoporous silicas described in U.S. Patent No. 6,592,764. Amongst these are the mesopourous silica materials that have an ordered, two-dimensional hexagonal (p6mm) honeycomb mesostructure, in particular the materials designated as SBA-15. These materials have porous structures with high Brunauer-Emmett-Teller (BET) surface areas that may be in the range of about 690 to about 1,040 m²/g, they may have pore volumes up to about 2.5 mL/g, they may have ultra large d(100) spacings in the range of about 7.45 - 45 nm, they may have pore sizes in the range of about 4.0 - 30 nm, and they may have silica wall thicknesses in the range of about 3 - 14 nm. Such materials can be prepared using amphiphilic block copolymers, in particular triblock polyoxyalkylenes as templating agents in acidic media followed by calcination at increased temperatures.

Further ordered mesoporous silicas that can be used are the mesoporous silicas described in WO 2009/133100, in particular the silica materials referred to as COK-12. These materials, structurally similar to the SBA-15 materials also have a 2-D hexagonally ordered mesostructure, have a slightly thicker silica wall, in particular a wall thickness of >4 nm, and have different silicate connectivity in the pore walls. The ratio of Q3 to Q4 silica in these materials, in particular in COK-12, may be less than about 0.65 and preferably less than about 0.60. The ratio of Q3 to Q4 silica can be measured by Si MAS NMR. Q3 silica is a silicon atom linked with three O-Si bridges and having one free hydroxyl group. A Q4 silica is a silicon atom linked with four O-Si bridges and having no free hydroxyl group. COK-12 materials can be obtained from the same starting materials as the SBA-15 materials, but using an amphiphilic block copolymer, in particular a triblock polyoxyalkylene, as templating agent in neutral or slightly acidic reaction conditions such as pH 5 - 8, or pH 5.5 - 7, or pH 6 - 7. Preferably a buffer is used to stabilize the pH within these ranges, for example a citrate/citric acid buffer. The triblock polyoxyalkylenes in particular are polyethylene glycol -polypropylene glycol - polyethylene glycol co-block polymers. For a more detailed description of the preparation of such materials reference is made to WO 2009/133100.

In one embodiment the ordered mesoporous silica for use in the invention is SBA-15 or COK-12. Of interest are SBA-15 or COK-12 mesoporous silicas having uniform sized cylindrical pores of about 4 - 14 nm, or about 5 - 14 nm, in particular about 6 - 14 nm, or about 6 - 12 nm. Their pore volume may be in the range from about 0.8 - 1.2 mL/g, or about 0.9 - 1.1 mL/g, and their specific surface area may be in the range from about 500 - 1000 m²/g, or from about 700 - 1000 m²/g. The wall thickness may be as specified above.

The present invention concerns ordered mesoporous silica materials that are unloaded or loaded with an active ingredient, the term "loaded" meaning that an active ingredient is adsorbed at the surface of the silica, including the surface within the pores of the silica. A major part of the active ingredient is incorporated in the pores of the silica material. Such silicas with adsorbed active ingredient are referred as "loaded silicas". The terms "loaded" and "incorporated" in this context are meant to be equivalent. "Unloaded" OMS materials do not contain an active ingredient adsorbed at their surface.

The OMS materials or unloaded granulates may be loaded with one or with two or more active ingredients.

The biologically active ingredient to be incorporated into the ordered mesoporous silica is sized such that it can fit into the cavities of the silica. In particular, one of its dimensions is smaller than the diameter of the pores, such as below 12 nm, or below 10 nm, or below 7 nm, or below 5 nm, or below 2 nm. In one embodiment, the active ingredient has a molecular weight in the range of 200 to 1,000 (daltons), in particular in the range of 200 to 800.

The biologically active ingredient for use in the invention may be very slightly soluble or practically insoluble in water or aqueous media, in particular in physiological aqueous media. According to the manual, Pharmaceutics (M.E. Aulton), any solvent solubility is defined as the amount of a solvent (g) required to solve 1 g of a compound, whereby the following solubility qualifications are defined: 10-30 g ("soluble"); 30-100 g ("sparingly soluble"); 100-1000 g ("slightly soluble"); 1000-10000 g ("very slightly soluble" or "poorly soluble") and more than 10000 g (practically insoluble).

The biologically active ingredient for use in the present invention may belong to the so-called BCS classes II and IV. The Biopharmaceutical Classification System (BCS) classifies drug substances into four classes: Class I- High Permeability, High Solubility; Class II- High Permeability, Low Solubility; Class III- Low Permeability, High Solubility; Class IV- Low Permeability, Low Solubility.

The solubility class boundary is based on the solubility of the highest dose strength in 250 ml or less of an immediate release ("IR") formulation of a drug in aqueous media over a pH range of 1.2 to 7.5 at 37°C. A drug substance has high solubility when the highest dose strength is soluble under these conditions, otherwise it has low solubility. A drug substance is considered highly permeable when the extent of absorption in humans is determined to be greater than 90% of an administered dose, otherwise it is considered to have low permeability.

BCS Class II drugs include, for example, anti-fungals, such as itraconazole, fluconazole, terconazole, ketoconazole, griseofulvin, and griseoverdin; anti-infectives such as sulfasalazine; anti-malaria drugs (e.g. atovaquone); immune system modulators (e.g. cyclosporin); cardiovascular drugs (e.g. digoxin and spironolactone); ibuprofen (analgesic); ritonavir, nevirapine, lopinavir (antiviral); clofazinine (leprostatic); diloxanide furoate (anti-amebic); glibenclamide (anti-diabetes); nifedipine (anti- anginal); spironolactone (diuretic); sterols or steroids such as danazol; carbamazepine; anti-virals such as acyclovir; antibiotics such as amoxicillin, tetracycline, or metronidazole; acid suppressants (H2 blockers including cimetidine, ranitidine, famotidine, and nizatidine; proton pump inhibitors including omeprazole, lansoprazole, rabeprazole, esomeprazole, and pantoprozole), mucolytic agents (megaldrate) .

Examples of BCS Class IV Drugs include acetazolamide, furosemide, tobramycin, cefuroxime, allopurinol, dapsone, doxycycline, paracetamol, nalidixic acid, clorothiazide, tobramycin, cyclosporin, tacrolimus, and paclitaxel.

Further examples of active ingredients that are poorly soluble in water include prostaglandines, e.g. prostaglandine E2, prostaglandine F2 and prostaglandine E1; cytotoxics, e.g. paclitaxel, doxorubicin, daunorubicin, epirubicin, idarubicin, zorubicin, mitoxantrone, amsacrine, vinblastine, vincristine, vindesine, dactiomycine, bleomycine; metallocenes, e.g. titanium metallocene dichloride; lipid-drug conjugates, e.g. diminazene stearate and diminazene oleate; anti-infectives such as clindamycin; antiparasitic drugs, e.g chloroquine, mefloquine, primaquine, vancomycin, vecuronium, pentamidine, metronidazole, nimorazole, tinidazole, atovaquone, buparvaquone, nifurtimoxe; anti-inflammatory drugs, e.g. methotrexate, azathioprine.

The term "active ingredient" also encompasses small antibody fragments. The latter include Fv" fragments, single-chain Fv (scFv) antibodies, antibody Fab fragments, antibody Fab' fragments, antibody fragments of heavy or light chain CDRs, or nanobodies. Also encompassed are small oligonucleic acid or peptide molecules such as aptamers, for example DNA aptamers, RNA aptamers or peptide aptamers.

The ordered mesoporous silica materials can be loaded with a pharmaceutically active ingredient by the solvent method, the incipient wetness method, or the melt method, which methods have been described in the prior art. The same methods can be used to load dry granulate prepared from unloaded OMS.

The release of a biologically active ingredient from the dry granulates of OMS materials loaded therewith may remain unchanged or may remain within acceptable boundaries as compared to the release from non-compacted OMS materials loaded with a biologically active ingredient, when tested with a standard pharmaceutical dissolution test. The term "within acceptable boundaries" means that release of biologically active ingredient is sufficient to exert the desired pharmacological or other effect. In one embodiment this term means that the quantity of biologically active ingredient from dry granulates of loaded OMS released after a period of e.g. about 60 minutes, or about 30 minutes, shows a 20% difference, or a 10% difference, or a 5% difference, when comparing drug release from non-granulated loaded OMS, wherein each % is w/w.

The pharmaceutically biologically active ingredient may be released from the dry granulate of the invention as well as from dosage forms prepared therefrom in a controlled fashion (controlled release). Preferably, the biologically active ingredient may be released from the dry granulate of the invention as well as from dosage forms prepared therefrom in a short period of time complying with immediate release. The term "immediate release" in this context means, for example, a release of at least 30%, or at least 40%, or at least 50%, or at least 60%, or at least 70%, or at least 80%, or at least 90%, of the drug under physiological conditions (pH, temperature), within a short period of time such as within 4 hours, or within 3 hours, or within 2 hours, such as within 60 minutes, or within 30 minutes, or within 20 minutes, or within 15 minutes. Upon conversion of active ingredient-loaded OMS into active ingredient-loaded granulate, the release characteristics and in particular the immediate release characteristics are maintained.

The weight percentage of biologically active ingredient released from the dry granules of the invention relative to the total amount of active ingredient in the dry granulate may be higher than 50% after 15 min, or higher than 60% after 30 min, or higher than 70% after 60 min, or higher than 80% after 120 min.

In a further aspect, the present invention concerns oral dosage forms in the form of tablets, obtained or obtainable by compression of the dry granulate of the invention. Such dosage forms may be prepared by compressing the dry granulate of the invention optionally in admixture with further excipients. The latter may comprise any of the excipients customarily used in the manufacture of tablets. In one embodiment, a combination of croscarmellose sodium and microcrystalline cellulose, and optional further excipients, can be added.

Various pressures can be used to prepare tablets, for example pressures in the range of 20 to 500 MPa, or in the range of 60 to 180 MPa, or in the range of 60 to 100 MPa.

In the embodiments where the solid dosage form is a tablet, prepared from a mixture of drug loaded granulate and a combination of croscarmellose sodium and microcrystalline cellulose, and optional further excipients such as lactose, the drug release from the thus-prepared tablets may remain unchanged or remain within acceptable boundaries as compared to drug release from the dry granulate from which the tablet is prepared. The term "within acceptable boundaries" is as described above and in particular is such that drug release from the tablet is sufficient to exert the desired pharmacological effect.

In still a further aspect, the present invention concerns dosage forms in the form of a capsule filled with free-flowing compacted powder of the invention. The capsule can be filled using procedures known in the pharmaceutical art.

In one embodiment, the excipient mixture to be compressed into a tablet and also in the tablet itself comprises from 20 to 100%, from 20 to 75%, or from 25 to 70%, or from 5 to 15% of dry granulate loaded with an active ingredient. In a further embodiment, the tablet may comprise, apart from the dry granulate, from 10 to 90%, or from 30 to 70%, or from 50 to 90%, or from 60 to 85%, of microcrystalline cellulose. In a further embodiment, the tablet may comprise, apart from the dry granulate, from 3 to 20% of croscarmellose sodium, or from 5 to 20%, or from 10 to 15%, of croscarmellose sodium. The tablet, apart from the dry granulate, may contain from 0 to 50%, or from 0-20%, or from 5 to 10%, of further excipients. All percentages in this paragraph are weight/weight.

In some embodiments the MCC in the excipient mixtures to prepare tablets, and hence in the tablets themselves, is silicified MCC, in particular where croscarmellose sodium is present in the formulations of this invention in low amounts, such as 3 to 10%, or 3-5%, the MCC in these formulations may be silicified MCC.

The excipient mixtures to prepare tablets may also contain optional excipients. These may comprise any of the ingredients customarily employed in the art such as diluents, binders, granulating agents, glidants (flow aids), lubricants; disintegrants, sweeteners, flavors, and pigments to make the tablets visually attractive. Examples of such excipients include hydroxypropylmethyl cellulose, crospovidone, magnesium stearate, lactose, and talcum.

The excipient mixtures to prepare tablets a mixture of drug-loaded granulate as specified herein, croscarmellose sodium, MCC, and/or lactose.

In one embodiment, the weight/weight ratio between MCC and Croscarmellose sodium in in the excipient mixtures to prepare tablets may be in the range of 5 : 1 to 15 : 1.

The following examples are meant to illustrate the present invention and should not be construed as limiting its scope.

### Example 1: Flowability of dry granules produced via slugging using a Gamlen™ Tablet Press

An ordered mesoporous silica material, prepared according to the procedures disclosed in WO 2009/133100, was used throughout the experiments described in the following examples. This material, referred to hereafter as OMS-7, had a mean pore diameter of about 7 nm, a total surface area of about 900 m²/g and a total pore volume of about 1.1 cm³/g. Three drugs (carbamazepine, fenofibrate and itraconazole) were first loaded onto the material OMS-7 at a drug loading of 30% via impregnation with a concentrated solution in methylene chloride. The drug-loaded OMS-7 powders were then blended with additional excipients to obtain the compositions summarized in Table 2. These powder blends were slugged at 35 MPa using a Gamlen™ Tablet Press equipped with a 6 mm, flat-face, cylindrical die, and subsequently milled using mortar and pestle and passed through a 900 µm sieve. The flowability of the granules was assessed via measurement of the Carr Index. The data in Table 2 indicate that the Carr Index of the granules was slightly lower or slightly higher than 20, indicative of "fair" or "passable" flowability, respectively, according the USP classification for flowabiltiy measured via the Carr Index (see Table 1).

**Table 2: Composition and flowability of dry granules produced via slugging using a Gamlen™ Tablet Press**

| Drug | Drug loading in OMS-7 (%) | Blend composition (%) | | | | Carr Index |
|---|---|---|---|---|---|---|
| | | Drug-loaded OMS-7 | Ceolus™ KG-1000 | Acdisol™ SD-711 | Mg stearate | |
| Carbamazepine | 30 | 84 | 10 | 5 | 1 | 20.8 |
| Fenofibrate | 30 | 84 | 10 | 5 | 1 | 19.1 |
| Itraconazole | 30 | 84 | 10 | 5 | 1 | 20.5 |

| | | | | | | |
|---|---|---|---|---|---|---|
| a Granules were prepared from slugs compacted at 35 MPa using a Gamlen™ Tablet Press equipped with a 6 mm, flat-face, cylindrical die. Slugs were milled using mortar and pestle and passed through a 900 µm sieve. b Bulk and tapped volumes used to calculate the Carr Index were determined as follows: 2 g of powder was poured gently into a 10 ml graduated cylinder using a funnel with an orifice diameter of 5 mm. The volume occupied by the freely settled powder bed was noted directly from the scale of the graduated cylinder. The tapped volume was obtained by mechanically tapping the graduated cylinder, and was recorded after no further volume reduction was observed following tapping. Ceolus™ KG-1000 is MCC (binder) and Acdisol™ SD-711 is croscarmellose sodium (disintegrant). | | | | | | |

### Example 2: Drug release from dry granules produced via slugging on a Gamlen™ Tablet Press.

The granules produced via slugging using the Gamlen™ Tablet Press (composition and slugging conditions provided in Table 2) were subjected to in vitro release experiments, and the release rate compared to that of the uncompressed loaded OMS-7 powders. All release profiles were recorded using a USP II (paddle) apparatus. Release of Itraconazole was recorded in 0.01 N HCl supplemented with 1% of sodium lauryl sulfate. A dose of 12 mg itraconazole was used for a volume of 100 ml of medium. Release of fenofibrate was recorded in 50 mM phosphate buffer supplemented with 25 mM of sodium lauryl sulfate. A dose of 33.5 mg fenofibrate was used for a volume of 500 ml of medium. ) Release of carbamazepine was recorded in 50 mM phosphate buffer supplemented with 0.5% of sodium lauryl sulfate. A dose of 12 mg carbamazepine was used for a volume of 100 ml of medium.
The release profiles are shown in Figure 1 A, B and C and show that the release rate from the slugs is only slightly decelerated when compared to the uncompressed powders.

### Example 3: Flowability of dry granules produced via slugging using a Korsch XP1 single punch press

The drug fenofibrate was loaded onto OMS-7 via impregnation with a concentrated solution in methylene chloride to obtain a drug loading of 30% w/w. The drug-loaded OMS-7 powder was then blended with additional excipients to obtain the compositions summarized in Table 3. The blends were compacted into slugs at 35 MPa using a Korsch™ XP1 single punch press equipped with a flat-face, bevel-edged, round-shaped punch. The slugs were gently crushed in a mortar and subsequently passed through a 900 µm sieve. The flowability of the obtained granules was assessed via measurement of the Carr Index.

**Table 3: Composition and flowability of dry granules produced via slugging using a Korsch™ XP1 single punch press**

| | Blend composition (%) | | | | |
|---|---|---|---|---|---|
| Drug | Drug-loaded OMS-7 | Ceolus™ KG-1000 | Acdisol™ SD-711 | Magnesium stearate | Carr Index^{b} |
| Fenofibrate | 84 | 10 | 5 | 1 | 20,0 |
| Fenofibrate | 74 | 20 | 5 | 1 | 20,0 |

| | | | | | |
|---|---|---|---|---|---|
| a Granules were prepared from slugs compacted at a targeting pressure of 35 MPa (the actual pressure varied between 31 -39 MPa) using a Korsch™ XP1 single punch press equipped with a flat-face, bevel-edged, round-shaped punch (19 mm diameter). The produced slugs were milled using mortar and pestle and passed through a 900 µm sieve. b Bulk and tapped densities used to calculate the Carr Index were measured using a Tap Density Tester TD1 (Sotax, Allschwil, Switzerland) equipped with a 25 mL graduated cylinder and operated at a tapping speed of 250 taps/min and a tap height of 3 mm. A mass of powder of approximately 5 g was used. | | | | | |

The data in Table 3 show that granules with "fair" flowability (Carr Index ≤ 20) could be produced via slugging using a pilot scale single punch press (Korsch XP1). The flowability of the granules produced from the 84:10:5:1 and 74:20:5:1 fenofibrate-loaded OMS-7 : Ceolus™ : Acdisol™ : Magnesium stearate blend was the same.

### Example 4: Flowability of drug-loaded OMS-7 dry granules produced via roller compaction

The drug fenofibrate was loaded onto OMS-7 via impregnation with a concentrated solution in methylene chloride to obtain a total drug loading of 30% w/w.

The fenofibrate-loaded OMS-7 was physically blended with additional excipients and subsequently compacted into ribbons using a roller compactor (Fitzpatrick Chilsonator™ IR 220). The composition of the powder blends and the pressure at which they were compacted are summarized in Table 4. The ribbons were milled using a knife rotor which was coupled to the roller compactor. Granules with particle size 355-520 µm were isolated via sieving, and assessed for flowability via measurement of the Carr Index. The values of the Carr Index of the uncompressed powder blend and the granules are also summarised in Table 4. The data in Table 4 indicate that the uncompressed powder blends have a Carr Index of 38 or higher, which is classified as "very, very poor" flowability, whereas the granules have "good" or "excellent" flowability according to the United States Pharmacopeia Classification.

**Table 4: Overview of composition of compacted powder blends, hydraulic pressures used for roller compaction and flowability of dry granules.**

| Run | A | B | C | D |
|---|---|---|---|---|
| Powder blend composition (%) | | | | |
| Fenofibrate-loaded OMS-7 | 60.0 | 80.0 | 60.0 | 80.0 |
| Supertab™ 21AN | 26.2 | 11.2 | 26.2 | 11.2 |
| Avicel™ pH 302 | 8.8 | 3.8 | - | - |
| Ceolus™ KG 1000 | - | - | 8.8 | 3.8 |
| Acdisol™ SD 711 | 5.0 | 5.0 | 5.0 | 5.0 |

| Hydraulic pressure | | | | |
|---|---|---|---|---|
| kN/cm | 2.1 | 4.2 | 2.1 | 4.2 |

| Carr Index^{a} (%) | | | | |
|---|---|---|---|---|
| Non-granulated powder blend | ND^{c} | 52.0 | 46.7 | 51.4 |
| Granules^{b} | 8.8 | 4.8 | 8.6 | 14.3 |

| | | | | |
|---|---|---|---|---|
| a Bulk and tapped volumes used to calculate the Carr Index were determined as follows: 5 g of powder was poured gently in a 25 ml graduated cylinder using a funnel with an orifice diameter of 5 mm. The volume occupied by the freely settled powder bed was noted directly from the scale of the graduated cylinder. The tapped volume was obtained by mechanically tapping the graduated cylinder, and was recorded after no further volume reduction was observed following tapping. b Granules were obtained by milling the compacted ribbons using a knife rotor coupled to the roller compactor, followed by isolation of the 355-520 µm particle size fraction. c ND: No data available Ceolus™ KG-1000 and Avicel™ pH302 are MCC (binders), Acdisol™ SD-711 is croscarmellose sodium (disintegrant) and Supertab™ 21AN is lactose (filler). | | | | |

### Example 5: Release rate from drug-loaded OMS-7 dry granules prepared via roller compaction

Granules were prepared from fenofibrate-loaded OMS-7 as described in example 4, Run C (see Table 4). The particle size fraction 355-520 µm was used. The release profile was recorded using a USP II (paddle) apparatus. Water supplemented with 25 mM sodium lauryl sulfate was used as release medium. A dose of 3.35 mg fenofibrate was dispersed in 100 ml of medium. The release profile is shown in Figure 2 (n=3; mean ± standard deviation).

The data in Figure 2 show that about 90% of the fenofibrate dose was released from the granules within 60 min.

## Claims

1. A dry granulate comprising from 50 % to 100% w/w of ordered mesoporous silica having a hexagonally-ordered two-dimensional array of uniform sized cylindrical pores of 4 to 14 nm; a pore volume in the range from 0.8 - 1.2 mL/g, and a specific surface area in the range from 500 - 1000 m²/g, wherein said ordered mesoporous silica is loaded with a biologically active ingredient having a molecular weight in the range of about 200 to about 1,000 daltons in particular in the range of about 200 to about 800, and optional excipients up to 100% w/w, said dry granulate having a Carr index that is equal or below 25.

2. The dry granulate according to claim 1, wherein the dry granulate comprises from 80 % to 100% w/w of ordered mesoporous silica loaded with a biologically active ingredient and optional excipients up to 100% w/w.

3. The dry granulate according to claim 1 or to claim 2, wherein the optional excipients are selected from one or more of a disintegrant, a binder, a filler and a lubricant.

4. The dry granulate according to claim 3, containing from 3 to 30 weight% of a binder.

5. The dry granulate according to claim 3 or claim 4, containing from 1 to 10 weight% of a disintegrant.

6. The dry granulate according to any of claims 1 to 5, containing from 0.5 to 2 weight% of a lubricant.

7. The dry granulate according to any of claims 1 to 6, wherein said biologically active ingredient is a poorly-soluble pharmaceutically active ingredient.

8. The dry granulate according to any of claims 1 to 7, wherein the ordered mesoporous silica is a hexagonally-ordered two-dimensional array of uniform sized cylindrical pores of 5 to 14 nm.

9. The dry granulate according to any of claims 1 to 8, wherein the ordered mesoporous silica is a hexagonally-ordered two-dimensional array of uniform sized cylindrical pores of 6 to 14 nm; a pore volume in the range from 0.9 - 1.1 mL/g, and a specific surface area in the range from 700 - 1000 m²/g.

10. A process for preparing a dry granulate as defined in any of claims 1 to 9, wherein a mixture of ordered mesoporous silica loaded with a biologically active ingredient and optional excipients is subjected to pressure that is in the range of 30 to 50 MPa.

11. The process according to claim 10, which comprises a step of compaction wherein the compaction is achieved by compression using dies thus forming a tablet, or by roller-compaction, optionally followed by milling of the compacted material.

12. The process according to claim 11, wherein the thus prepared dry granulate is sieved into a desired particle size range.

13. A solid dosage form comprising the dry granulate according to any of claims 1 to 9.

14. The solid dosage form according to claim 13 in the form of a tablet.

15. The dosage form according to claim 13, which is a capsule filled with the dry granulate according to any of claims 1 to 9.

## Patentansprüche

1. Trockengranulat, umfassend 50 Gew.-% bis 100 Gew.-% geordnetes mesoporöses Siliciumdioxid mit einer hexagonal geordneten zweidimensionalen Anordnung zylindrischer Poren mit einer einheitlichen Größe von 4 bis 14 nm, einem Porenvolumen im Bereich von 0,8 - 1,2 ml/g und einer spezifischen Oberfläche im Bereich von 500 - 1000 m²/g, wobei das geordnete mesoporöse Siliciumdioxid mit einem biologischen Wirkstoff mit einem Molekulargewicht im Bereich von etwa 200 bis etwa 1 000 Dalton, insbesondere im Bereich von etwa 200 bis etwa 800 Dalton, und gegebenenfalls Exzipienten bis zu 100 Gew.-% beladen ist, wobei das Trockengranulat einen Carr-Index gleich oder kleiner als 25 aufweist.

2. Trockengranulat nach Anspruch 1, wobei das Trockengranulat 80 Gew.-% bis 100 Gew.-% geordnetes mesoporöses Siliciumdioxid beladen mit einem biologischen Wirkstoff und gegebenenfalls Exzipienten bis zu 100 Gew.-% umfasst.

3. Trockengranulat nach Anspruch 1 oder Anspruch 2, wobei die fakultativen Exzipienten aus Sprengmitteln, Bindemitteln, Füllstoffen und/oder Schmiermitteln ausgewählt sind.

4. Trockengranulat nach Anspruch 3, welches 3 bis 30 Gew.-% an Bindemittel enthält.

5. Trockengranulat nach Anspruch 3 oder Anspruch 4, welches 1 bis 10 Gew.-% an Sprengmittel enthält.

6. Trockengranulat nach einem der Ansprüche 1 bis 5, welches 0,5 bis 2 Gew.-% eines Schmiermittels enthält.

7. Trockengranulat nach einem der Ansprüche 1 bis 6, wobei es sich bei dem biologischen Wirkstoff um einen schlecht löslichen pharmazeutischen Wirkstoff handelt.

8. Trockengranulat nach einem der Ansprüche 1 bis 7, wobei es sich bei dem geordneten mesoporösen Siliciumdioxid um eine hexagonal geordnete zweidimensionale Anordnung zylindrischer Poren mit einer einheitlichen Größe von 5 bis 14 nm handelt.

9. Trockengranulat nach einem der Ansprüche 1 bis 8, wobei es sich bei dem geordneten mesoporösen Siliciumdioxid um eine hexagonal geordnete zweidimensionale Anordnung zylindrischer Poren mit einer einheitlichen Größe von 6 bis 14 nm, einem Porenvolumen im Bereich von 0,9 - 1,1 ml/g und einer spezifischen Oberfläche im Bereich von 700 - 1000 m²/g handelt.

10. Verfahren zur Herstellung eines wie in einem der Ansprüche 1 bis 9 definierten Trockengranulats, bei dem man eine Mischung von mit einem biologischen Wirkstoff und gegebenenfalls Exzipienten beladenen geordneten mesoporösen Siliciumdioxid einem Druck im Bereich von 30 bis 50 MPa aussetzt.

11. Verfahren nach Anspruch 10, welches einen Verdichtungsschritt umfasst, wobei die Verdichtung durch Kompression unter Verwendung einer Pressform unter Bildung einer Tablette oder durch Walzenverdichtung erfolgt, gegebenenfalls mit anschließendem Mahlen des verdichteten Materials.

12. Verfahren nach Anspruch 11, wobei das auf diese Weise hergestellte Trockengranulat gesiebt wird, so dass man den gewünschten Teilchengrößenbereich erhält.

13. Feste Dosierungsform, umfassend das Trockengranulat nach einem der Ansprüche 1 bis 9.

14. Feste Dosierungsform nach Anspruch 13 in Form einer Tablette.

15. Dosierungsform nach Anspruch 13, bei der es sich um eine mit dem Trockengranulat nach einem der Ansprüche 1 bis 9 gefüllte Kapsel handelt.

## Revendications

1. Matière granulaire sèche comprenant de 50 % à 100 % p/p de silice mésoporeuse ordonnée ayant un réseau bidimensionnel ordonné de manière hexagonale de pores cylindriques de taille uniforme de 4 à 14 nm ; un volume poreux dans la plage de 0,8-1,2 ml/g et une surface spécifique dans la plage de 500-1000 m²/g, dans laquelle ladite silice mésoporeuse ordonnée est chargée d'un principe biologiquement actif ayant une masse moléculaire dans la plage d'environ 200 à environ 1000 daltons, en particulier dans la plage d'environ 200 à environ 800, et des excipients facultatifs complétant à 100 % p/p, ladite matière granulaire sèche ayant un indice de Carr qui est inférieur ou égal à 25.

2. Matière granulaire sèche selon la revendication 1, la matière granulaire sèche comprenant de 80 % à 100 % p/p de silice mésoporeuse ordonnée chargée d'un principe biologiquement actif et des excipients facultatifs complétant à 100 % p/p.

3. Matière granulaire sèche selon la revendication 1 ou la revendication 2, dans laquelle les excipients facultatifs sont choisis parmi un délitant, un liant, une charge et/ou un lubrifiant.

4. Matière granulaire sèche selon la revendication 3, contenant de 3 à 30 % en poids d'un liant.

5. Matière granulaire sèche selon la revendication 3 ou la revendication 4, contenant de 1 à 10 % en poids d'un délitant.

6. Matière granulaire sèche selon l'une quelconque des revendications 1 à 5, contenant de 0,5 à 2 % en poids d'un lubrifiant.

7. Matière granulaire sèche selon l'une quelconque des revendications 1 à 6, dans laquelle ledit principe biologiquement actif est un principe pharmaceutiquement actif faiblement soluble.

8. Matière granulaire sèche selon l'une quelconque des revendications 1 à 7, dans laquelle la silice mésoporeuse ordonnée est un réseau bidimensionnel ordonné de manière hexagonale de pores cylindriques de taille uniforme de 5 à 14 nm.

9. Matière granulaire sèche selon l'une quelconque des revendications 1 à 8, dans laquelle la silice mésoporeuse ordonnée est un réseau bidimensionnel ordonné de manière hexagonale de pores cylindriques de taille uniforme de 6 à 14 nm ; un volume poreux dans la plage de 0,9-1,1 ml/g et une surface spécifique dans la plage de 700-1000 m²/g.

10. Procédé pour la préparation d'une matière granulaire sèche telle que définie dans l'une quelconque des revendications 1 à 9, dans lequel un mélange de silice mésoporeuse ordonnée chargée d'un principe biologiquement actif et d'excipients facultatifs est soumis à de la pression qui est dans la plage de 30 à 50 MPa.

11. Procédé selon la revendication 10, qui comprend une étape de compactage dans lequel le compactage est réalisé par compression à l'aide de matrices ce qui forme ainsi un comprimé, ou par compactage au rouleau, éventuellement suivie du broyage du matériau compacté.

12. Procédé selon la revendication 11, dans lequel la matière granulaire sèche ainsi préparée est criblée à une plage granulométrique souhaitée.

13. Forme galénique solide comprenant la matière granulaire sèche selon l'une quelconque des revendications 1 à 9.

14. Forme galénique solide selon la revendication 13 sous la forme d'un comprimé.

15. Forme galénique solide selon la revendication 13, qui est une capsule remplie de la matière granulaire sèche selon l'une quelconque des revendications 1 à 9.
